(19) Europäisches Patentamt
European Patent Office
Office européen des brevets

(11) EP 1 928 441 B1

(12) # EUROPÄISCHE PATENTSCHRIFT

(45) Veröffentlichungstag und Bekanntmachung des Hinweises auf die Patenterteilung:
**27.02.2013 Patentblatt 2013/09**

(21) Anmeldenummer: **06805729.8**

(22) Anmeldetag: **15.09.2006**

(51) Int Cl.:
***A61K 9/20*** (2006.01) ***A61K 9/28*** (2006.01)
***A61K 31/133*** (2006.01) ***A61K 47/38*** (2006.01)
***A61K 31/00*** (2006.01)

(86) Internationale Anmeldenummer:
**PCT/EP2006/008988**

(87) Internationale Veröffentlichungsnummer:
**WO 2007/031326 (22.03.2007 Gazette 2007/12)**

(54) **RETARDFORMULIERUNG VON 3-(2-DIMETHYLAMINOMETHYL-CYCLOHEXYL)-PHENOL**

RETARDFORMULATION COMPRISING 3-(2-DIMETHYLAMINOMETHYLCYCLOHEXYL)PHENOL

FORMULATION À EFFET RETARD COMPRENANT DU 3-(2-DIMÉTHYLEAMINOMÉTHYLECYCLOHEXYLE)PHENOL

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**HR**

(30) Priorität: **15.09.2005 DE 102005044212**

(43) Veröffentlichungstag der Anmeldung:
**11.06.2008 Patentblatt 2008/24**

(73) Patentinhaber: **Grünenthal GmbH**
**52078 Aachen (DE)**

(72) Erfinder:
- **JUNG, Tobias**
**79588 Efringen-Kirchen (DE)**
- **BARTHOLOMÄUS, Johannes**
**52080 Aachen (DE)**

(74) Vertreter: **Bülle, Jan et al**
**Kutzenberger & Wolff**
**Patentanwaltssozietät**
**Theodor-Heuss-Ring 23**
**50668 Köln (DE)**

(56) Entgegenhaltungen:
**WO-A-02/067651** **WO-A2-2005/009329**
**DE-A1- 4 329 794** **DE-A1- 19 525 137**
**US-A- 5 601 842**

- **BARTHOLOMÄUS JOHANNES: "[Situational administration of analgesics. Modern dosage forms for opioids]" PHARMAZIE IN UNSERER ZEIT 2002, Bd. 31, Nr. 1, 2002, Seiten 74-81, XP007901842 ISSN: 0048-3664**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 928 441 B1

## Beschreibung

Die Erfindung betrifft eine pharmazeutische Darreichungsform mit kontrollierter Freisetzung von 3-(2-Dimethylamino-methyl-cyclohexyl)-phenol, vorzugsweise des (1R,2R)-Stereoisomers, oder von einem seiner pharmazeutisch verträglichen Salze.

3-(2-Dimethylaminomethyl-cyclohexyl)-phenol ist im Stand der Technik bekannt. Es handelt sich um einen oral applizierbaren, analgetisch wirksamen Arzneistoff (vgl. z.B. DE-A 195 25 137, WO 02/43712 WO 02/67916 und WO 02/067651).

WO 2005/009329, US 5,601,842 und DE 43 29 794 offenbaren pharmazeutische Darreichungsformen zür verzögehen Freisetzung von Wirkstoffen, welche in Polymerization eingebettet sind.

Wegen der zwei Chiralitätszentren kommt 3-(2-Dimethylaminomethyl-cyclohexyl)-phenol in Form vierer Stereoisomere (zweier Enantiomerenpaare) vor, nämlich als (1R,2R)-, (1 S,2S)-, (1R,2S)- und (1S,2R)-3-(2-Dimethylaminomethyl-cyclohexyl)-phenol. Diese vier Stereoisomere haben die folgende Struktur

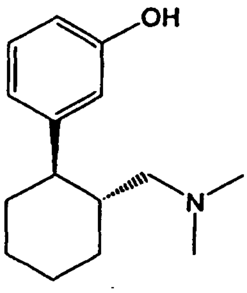
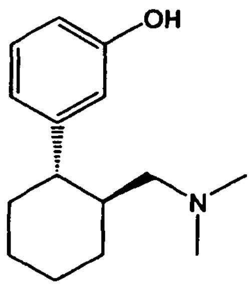
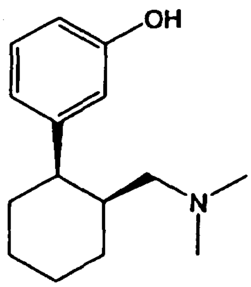
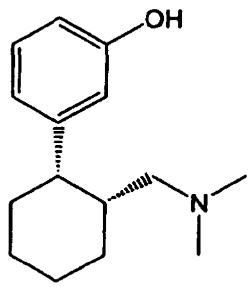

(1R,2R)

Herkömmliche Darreichungsformen für die orale Verabreichung führen bei 3-(2-Dimethylaminomethyl-cyclohexyl)-phenol zu einer raschen Freisetzung der gesamten Wirkstoffdosis im Gastrointestinaltrakt, so dass die analgetische Wirkung schnell einsetzt. Als Folge davon erfordert die Behandlung starker chronischer Schmerzen mit 3-(2-Dimethylaminomethyl-cyclohexyl)-phenol bislang die Verabreichung des Arzneimittels in relativ kurzen Zeitintervallen, beispielsweise vier- bis sechsmal täglich, um so eine ausreichende Wirkstoffkonzentration im Blutplasma des Patienten über einen Zeitraum von 24 h hinweg zu gewährleisten.

Die Notwendigkeit einer häufigen Dosierung führt jedoch leicht zu Fehlern bei der Einnahme sowie zu unerwünschten Schwankungen der Plasmakonzentration, was der *Compliance* und dem therapeutischen Nutzen abträglich ist, insbesondere bei der Behandlung chronischer Schmerzzustände. Darüber hinaus ist es bekannt, dass bei herkömmlichen Darreichungsformen die orale Verabreichung von 3-(2-Dimethylaminomethyl-cyclohexyl)-phenol zu Nebenwirkungen führen kann, insbesondere zu Übelkeit und Erbrechen.

Es ist bekannt, Darreichungsformen mit retardierter Freisetzung der darin enthaltenen Wirkstoffe bereitzustellen, um eine kontinuierliche Freisetzung des Wirkstoffs über einen längeren Zeitraum zu gewährleisten.

Im Stand der Technik sind Retardformulierungen für eine große Zahl von Wirkstoffen bekannt. Üblicherweise wird die Retardierung durch geeignete Überzüge und/oder durch die Einbettung des Wirkstoffs in eine die Freisetzung steuernde Matrix verwirklicht.

Bei Überzugsretardierungen wird ein wirkstoffhaltiger Kern mit einem die Freisetzung des Wirkstoffs verzögernden Überzug aus hydrophilen und/oder hydrophoben Polymeren versehen. Bei Matrixretardierungen ist der Wirkstoff in eine Polymermatrix eingebettet, welche die Freisetzung des Wirkstoffs steuert.

Um ein bestimmtes Freisetzungsprofil für einen Wirkstoff zu erhalten, ist es jedoch nicht ohne weiteres möglich, ausgehend von einer im Stand der Technik bekannten Zusammensetzung mit dem gewünschten Freisetzungsprofil den darin enthaltenen Wirkstoff einfach auszutauschen. Vielmehr müssen in jedem Einzelfall die individuellen physikalischen und chemischen Eigenschaften des jeweiligen Wirkstoffs berücksichtigt werden. So können zahlreiche individuelle Eigenschaften eines Wirkstoffs einen erheblichen Einfluss auf sein Freisetzungsprofil haben. Für dieses spezifische Freisetzungsverhalten eines Wirkstoffs können beispielsweise dessen zu verabreichende Dosis, Partikelgröße, Partikelform, Härte, Hygroskopizität, Löslichkeit, Abhängigkeit der Löslichkeit vom pH-Wert, Hydrophilie/Lipophilie, Säurestärke/Basizität, etc. verantwortlich sein.

Der Erfindung liegt die Aufgabe zugrunde, eine pharmazeutische Formulierung von 3-(2-Dimethylaminomethyl-cyclohexyl)-phenol, vorzugsweise seines (1R,2R)-Stereo-isomers bzw. eines seiner pharmazeutisch verträglichen Salze, bereitzustellen, welche Vorteile gegenüber den Formulierungen des Standes der Technik aufweist.

So sollte die Darreichungsform pharmakologisch wirksame Plasmakonzentrationen des Wirkstoffs 3-(2-Dimethylaminomethyl-cyclohexyl)-phenol über einen längeren Zeitraum, vorzugsweise für wenigstens 12 h, gewährleisten

(kontrollierte Freisetzung) und dabei durch ein möglichst geringes Nebenwirkungsspektrum gekennzeichnet sein, insbesondere im Hinblick auf Übelkeit und/oder Erbrechen. Darüber hinaus sollte sich das pharmakokinetische Verhalten in weiten Teilen vom pharmakokinetischen Verhalten einer Vergleichsformulierung ohne kontrollierte Freisetzung (Wirkstofflösung, Saft, *immediate release*) unterscheiden.

Diese Aufgabe wird durch den Gegenstand der Patentansprüche gelöst.

Es wurde überraschend gefunden, dass eine Darreichungsform des Wirkstoffs 3-(2-Dimethylaminomethyl-cyclohexyl)-phenol oder eines seiner pharmazeutisch verträglichen Salze hergestellt werden kann, welche den Wirkstoff kontrolliert freisetzt und dabei Vorteile gegenüber den Darreichungsformen des Standes der Technik aufweist.

Die Erfindung betrifft eine Darreichungsform zur kontrollierten Freisetzung des Wirkstoffs 3-(2-Dimethylaminomethyl-cyclohexyl)-phenol oder eines seiner pharmazeutisch verträglichen Salze, welche

- *in vitro,* gemessen gemäß Europäischem Arzneibuch mit einer Blattrührapparatur in Puffer bei einem pH-Wert von 6,8, einer Temperatur von 37°C und 75 U/min,

    - nach 0,5 Stunden 9,0 bis 31 Gew.-%,
    - nach 1 Stunde 20 bis 48 Gew.-%,
    - nach 2 Stunden 31 bis 65 Gew.-%,
    - nach 3 Stunden 42 bis 76 Gew.-%,
    - nach 4 Stunden 50 bis 83 Gew.-%,
    - nach 6 Stunden 60 bis 94 Gew.-%,
    - nach 8 Stunden 71 bis 99 Gew.-%,
    - nach 12 Stunden mehr als 79 Gew.-%,

    des ursprünglich in der Darreichungsform enthaltenen Wirkstoffs freisetzt,

- eine Polymermatrix umfasst, welche auf einem Celluloseether oder Celluloseester basiert, der bei einer Konzentration von 2,0 Gew.-% in einer wässrigen Lösung bei 20°C eine Viskosität im Bereich von 3.000 bis 150.000 mPa·s aufweist,

wobei zumindest ein Teil des Wirkstoffs in der Polymermatrix eingebettet ist,
wobei der Gewichtsanteil der Polymermatrix 5 bis 85 Gew.-%, bezogen auf das Gesamtgewicht der Darreichungsform, beträgt, und
wobei der Celluloseether bzw. Celluloseester ausgewählt ist aus der Gruppe bestehend aus Methylcellulose, Ethylcellulose, Hydroxyethylcellulose, Hydroxypropylcellulose, Carboxymethylcellulose und Hydroxypropylmethylcellulose.

Besonders bevorzugte Freisetzungsprofile der erfindungsgemäßen Darreichungsform (Nr. 1 bis Nr. 4) sind in nachfolgender Tabelle zusammengestellt:

| nach [h] | 1 | 2 | 3 | 4 |
|---|---|---|---|---|
| | Gew.-% | Gew.-% | Gew.-% | Gew.-% |
| 0,5 | 11-30 | 13-30 | 15-29 | 17-28 |
| 1 | 22-46 | 24-44 | 27-42 | 30-40 |
| 2 | 33-62 | 36-60 | 39-58 | 42-56 |
| 3 | 45-73 | 48-71 | 50-69 | 52-67 |
| 4 | 53-81 | 55-79 | 58-77 | 60-75 |
| 6 | 63-92 | 66-90 | 69-88 | 72-86 |
| 8 | 74-98 | 76-98 | 78-97 | 80-97 |
| 12 | >82 | >84 | >86 | >88 |

Die erfindungsgemäßen Darreichungsform setzt den Wirkstoff 3-(2-Dimethylaminomethyl-cyclohexyl)-phenol, vorzugsweise nach oraler Verabreichung, verzögert frei und eignet sich somit für eine Verabreichung im Abstand von mindestens 12 h. Die erfindungsgemäße Darreichungsform erlaubt demnach eine Schmerztherapie, in deren Verlauf der Wirkstoff 3-(2-Dimethylaminomethyl-cyclohexyl)-phenol nur noch einmal täglich, z.B. im Abstand von 24 h, oder zweimal täglich, vorzugsweise im Abstand von 12 h, verabreicht wird, um eine ausreichende Plasmakonzentration des

Wirkstoffs zu gewährleisten.

Dabei wurde überraschend gefunden, dass im Vergleich zu herkömmlichen Darreichungsformen zur oralen Verabreichung von 3-(2-Dimethylaminomethyl-cyclohexyl)-phenol Nebenwirkungen, insbesondere Übelkeit und/oder Erbrechen, signifikant reduziert werden können. Dies hat den Vorteil, dass sich die therapeutische Breite (Verhältnis der therapeutischen zur toxischen Wirkstoffdosis) von 3-(2-Dimethylaminomethyl-cyclohexyl)-phenol erhöht, wodurch u.a. die Wirkstoffdosis und damit auch die therapeutische Wirksamkeit erhöht werden kann.

Figur 1 zeigt mittlere Plasmakonzentrationen des Wirkstoffs 3-(2-Dimethylaminomethyl-cyclohexyl)-phenol nach oraler Verabreichung erfindungsgemäßer Darreichungsformen mit kontrollierter Freisetzung PR (A), und PR (B) sowie von Refereuzormintierüng PR (C) im Vergleich zu einer herkömmlichen Darreichungsform (Vergleichsformulierung ohne kontrollierte Freisetzung, Wirkstofflösung, Saft, IR). In Figur 1A ist die Ordinate linear, in Figur 1 B logarithmisch skaliert.

Bei einer herkömmlichen Darreichungsform ohne kontrollierte Freisetzung (*immediate release* IR, Wirkstofflösung, Saft) ist gewöhnlich davon auszugehen, dass der Verlauf des Plasmakonzentrations-Zeit-Diagramms recht bald nach der Verabreichung im Wesentlichen nur durch die Kinetik der Metabolisierung und Ausscheidung des Wirkstoffs aus dem Organismus bestimmt wird. Diese Kinetik ist von Wirkstoff zu Wirkstoff verschieden (Stoffkonstante), jedoch praktisch unabhängig von der Galenik der Darreichungsform.

Im Unterschied dazu ist bei einer herkömmlichen Darreichungsform mit kontrollierter Freisetzung gewöhnlich davon auszugehen, dass die Galenik der Darreichungsform den Verlauf des Plasmakonzentrations-Zeit-Diagramms solange beeinflusst, wie noch Wirkstoff aus der Darreichungsform freigesetzt wird. Während dieses Zeitintervalls überlagern sich dann die Kinetik der Nachlieferung des Wirkstoffs aus der Darreichungsform und die Kinetik der Metabolisierung und Ausscheidung des bereits freigesetzten Wirkstoffs. Ab einem gewissen Zeitpunkt ist jedoch die Freisetzung des Wirkstoffs aus der Darreichungsform soweit fortgeschritten, dass kaum noch Wirkstoff nachgeliefert wird. Die Kinetik der Nachlieferung des Wirkstoffs aus der Darreichungsform hat dann auf den Verlauf des Plasmakonzentrations-Zeit-Diagramms allenfalls noch geringen Einfluss. Vielmehr wird in dieser Phase der Verlauf des Plasmakonzentrations-Zeit-Diagramms im Wesentlichen nur noch durch die Kinetik der Metabolisierung und Ausscheidung des Wirkstoffs aus dem Organismus dominiert - es findet demnach während dieses Zeitintervalls eine Annäherung des Plasmakonzentrations-Zeit-Diagramms an den Verlauf statt, welcher bei Darreichungsformen ohne kontrollierte Freisetzung beobachtet wird. Wie jedoch aus Figur 1 hervorgeht, ist bei den erfindungsgemäßen Darreichungsformen (PR (A), und PR (B) mitunter auch noch viele Stunden nach der Verabreichung der Verlauf des Plasmakonzentrations-Zeit-Diagramms nicht an den Verlauf des Plasmakonzentrations-Zeit-Diagramms einer Vergleichsformulierung ohne kontrollierte Freisetzung (Wirkstofflösung, Saft, IR) angenähert. Wie insbesondere aus Figur 1 B hervorgeht, sind nämlich die Steigungen im terminalen Verlauf des Plasmakonzentrations-Zeit-Diagramms bei den erfindungsgemäßen Darreichungsformen mit kontrollierter Freisetzung (PR (A), und PR (B) auch noch viele Stunden nach der Verabreichung deutlich von der Steigung der Vergleichsformulierung ohne kontrollierte Freisetzung verschieden (die terminalen Ausläufe der Kurven verlaufen nicht parallel, sondern als Kurvenschar).

In der terminalen Eliminationsphase im Plasmakonzentrations-Zeit-Diagramm ist bei logarithmischer linearer Regression die negative Steigung der Regressionsgeraden definiert als die Geschwindigkeitskonstante $\lambda_z$. Das überraschende Verhalten der erfindungsgemäßen Darreichungsform mit kontrollierter Freisetzung im Vergleich zu einer herkömmlichen Darreichungsform ohne kontrollierte Freisetzung kann daher durch $\lambda_z$ bzw. die davon abgeleitete Halbwertszeit nachgewiesen werden.

Dieses überraschende Verhalten der erfindungsgemäßen Darreichungsform hat den Vorteil, dass sie *in vivo* auch noch zu Zeitpunkten eine retardierende Wirkung auf die Plasmakonzentration des Wirkstoffs ausübt, zu denen auf Grundlage der *in vitro* Freisetzungsdaten keine signifikante Retardierung mehr zu erwarten wäre. Die Retardierung *in vivo* (gemessene Plasmakonzentration) ist demnach gegenüber der Retardierung *in vitro* (gemessene Freisetzungswerte) verstärkt; sie wirkt sich überproportional aus. Damit wird die zu erwartende Wirkdauer verlängert und die Verträglichkeit weiter verbessert im Vergleich zu der zu erwartenden Situation ohne diesen Effekt *in vivo.*

Bei der "kontrollierten" Freisetzung des Wirkstoffs im Sinne der Beschreibung kann es sich - innerhalb der erfindungsgemäßen Grenzen - um eine verzögerte Freisetzung *(extended release, delayed release*), eine gestaffelte Freisetzung (*repeat action release*), eine hinhaltende Freisetzung *(prolonged release*) oder eine gleichmäßig hinhaltende Freisetzung (*sustained release*) handeln. Eine sofortige Wirkstofffreisetzung (*immediate release*), wie sie beispielsweise mit Hilfe einer Wirkstofflösung erreicht wird, ist nicht als kontrollierte Freisetzung des Wirkstoffs im Sinne der Beschreibung aufzufassen.

Die erfindungsgemäße Darreichungsform umfasst eine Polymermatrix, welche wenigstens einen Teil des in der Darreichungsform insgesamt enthaltenen Wirkstoffs verzögert freisetzt (Matrixretardierung). Dazu liegt zumindest dieser Teil des Wirkstoffs, vorzugsweise (1R,2R)-3-(2-Dimethylaminomethyl-cyclohexyl)-phenol oder eines seiner pharmazeutisch verträglichen Salze, in der Polymermatrix eingebettet vor.

In einer bevorzugten Ausführungsform der erfindungsgemäßen Darreichungsform umfasst sie zusätzlich einen Filmüberzug, welcher vorzugsweise wenigstens einen Teil des in der Darreichungsform insgesamt enthaltenen Wirkstoffs

verzögert freisetzt (Überzugsretardierung).

So ist es möglich, eine Matrixretardierung mit einer Überzugsretardierung zu kombinieren. Erfindungsgemäß bevorzugt erfolgt die Retardierung der Freisetzung des Wirkstoffs jedoch im Wesentlichen ausschließlich durch die Polymermatrix.

Die erfindungsgemäße Darreichungsform umfasst eine Polymermatrix, in welcher zumindest ein Teil des Wirkstoffs, vorzugsweise (1R,2R)-3-(2-Dimethylaminomethyl-cyclohexyl)-phenol oder eines seiner pharmazeutisch verträglichen Salze, eingebettet ist, wobei die Polymermatrix auf einem Celluloseether oder Celluloseester basiert, welcher bei einer Konzentration von 2,0 Gew.-% in einer wässrigen Lösung bei 20°C eine Viskosität, vorzugsweise bestimmt durch Kapillar-Viskosimetrie nach europäischem Arzneibuch, im Bereich von 3.000 bis 150.000 mPa·s, bevorzugt 5.000 bis 145.000 mPa·s, bevorzugter 10.000 bis 140.000 mPa·s, noch bevorzugter 25.000 bis 135.000 mPa·s, am bevorzugtesten 50.000 bis 130.000 mPa·s und insbesondere 80.000 bis 120.000 mPa·s aufweist.

Die Polymermatrix umfast zumindest einen Celluloseether oder Celluloseester ausgewählt aus der Gruppe bestehend aus Methylcellulose (MC), Ethylcellulose (EC), Hydroxyethylcellulose (HEC), Hydroxypropylcellulose (HPC), Carboxymethylcellulose (CMC) und Hydroxypropylmethylcellulose (HPMC). Am meisten bevorzugt sind HPMCs mit einer Viskosität von ca. 100.000 mPa·s, gemessen in einer 2 Gew.-%igen wässrigen Lösung bei 20°C. Zusätzlich können auch Polyethylenglykole (PEG) mit einem Gehalt von 0 bis 60 Gew.-% in der Matrix enthalten sein.

Der Gewichtsanteil der Polymermatrix liegt im Bereich von 5,0 bis 85 Gew.-%, bevorzugt von 10 bis 50 Gew.-% und am bevorzugtesten von 25 bis 45 Gew.-%, bezogen auf das Gesamtgewicht der Darreichungsform. Dabei werden bei der Bestimmung des Gewichtsanteils bevorzugt nur solche Polymere der erfindungsgemäßen Darreichungsform berücksichtigt, welche eine Matrix bilden, in die zumindest ein Teil des Wirkstoffs eingebettet ist. Übliche polymere Hilfsstoffe, wie beispielsweise mikrokristalline Cellulose, gehen hingegen nicht in die Bestimmung ein, wenn sie praktisch nicht an der Matrixbildung beteiligt sind.

In einer bevorzugten Ausführungsform der erfindungsgemäßen Darreichungsform liegt der Gewichtsanteil des Wirkstoffs, vorzugsweise (1R,2R)-3-(2-Dimethylaminomethyl-cyclohexyl)-phenol oder eines seiner pharmazeutisch verträglichen Salze, im Bereich von 0,5 bis 85 Gew.-%, bevorzugter von 5,0 bis 50 Gew.-% und am bevorzugtesten von 15 bis 35 Gew.-%, bezogen auf das Gesamtgewicht der Darreichungsform.

In den erfindungsgemäßen Darreichungsformen liegt der Wirkstoffgehalt vorzugsweise zwischen 0,5 und 85 Gew.-% und der Gehalt der Polymermatrix zwischen 8,0 und 40 Gew.-%. Besonders bevorzugt sind Darreichungsformen mit einem Wirkstoffgehalt zwischen 3,0 und 70 Gew.-%, insbesondere zwischen 8,0 und 66 Gew.-%, und einem Gehalt der Polymermatrix zwischen 10 und 35 Gew.-%, insbesondere zwischen 10 und 30 Gew.-%, bezogen auf das Gesamtgewicht der pharmazeutischen Zusammensetzung.

Bevorzugt liegt das relative Gewichtsverhältnis der Polymermatrix zum Wirkstoff, vorzugsweise zu (1R,2R)-3-(2-Dimethylaminomethyl-cyclohexyl)-phenol oder zu einem seiner pharmazeutisch verträglichen Salze, im Bereich von 3:1 bis 1:10, bevorzugter von 2,5:1 bis 1:8, noch bevorzugter von 2,2:1 bis 1:5 und am bevorzugtesten von 2:1 bis 1:2.

In einer besonders bevorzugten Ausführungsform der erfindungsgemäßen Darreichungsform umfasst die Polymermatrix Hydroxypropylmethylcellulose, welche bei einer Konzentration von 2,0 Gew.-% in einer wässrigen Lösung bei 20°C eine Viskosität, vorzugsweise bestimmt durch Kapillar-Viskosimetrie nach europäischem Arzneibuch, im Bereich von 50.000 bis 130.000 mPa·s aufweist, wobei gleichzeitig der Gewichtsanteil der Hydroxypropylmethylcellulose im Bereich von 15 bis 35 Gew.-% liegt, bezogen auf das Gesamtgewicht der Darreichungsform.

In den erfindungsgemäßen pharmazeutischen Darreichungsformen können ferner als weitere Bestandteile pharmazeutisch gebräuchliche Hilfsstoffe, wie z.B.

- Füllmittel, beispielsweise Laktose, mikrokristalline Cellulose (MCC) oder Calciumhydrogenphosphat, und/oder
- Gleit-, Schmier- und Fließregulierungsmittel, beispielsweise Talkum, Magnesiumstearat, Stearinsäure und/oder hochdisperses Siliciumdioxid,

enthalten sein, wobei deren Gesamtgehalt in der Tablette vorzugsweise zwischen 0 und 80 Gew.-%, bevorzugter zwischen 5,0 und 65 Gew.-% liegt.

Derartige Hilfsstoffe sind dem Fachmann bekannt. In diesem Zusammenhang kann beispielsweise vollumfänglich verwiesen werden auf H.P. Fiedler, Lexikon der Hilfsstoffe für Pharmazie, Kosmetik und angrenzende technische Gebiete, Editio Cantor Aulendorff, 2002.

Bevorzugt enthält die erfindungsgemäße Darreichungsform zumindest ein Füllmittel, wobei das relative Gewichtsverhältnis des Füllmittels oder der Summe aller Füllmittel zur Polymermatrix kleiner als 6:1 ist, bevorzugter im Bereich von 5:1 bis 1:2, noch bevorzugter von 4:1 bis 1:1,5 und am bevorzugtesten von 3:1 bis 1:1 liegt.

In einer bevorzugten Ausführungsform der erfindungsgemäßen Darreichungsform enthält sie ein Füllmittel ausgewählt aus der Gruppe bestehend aus

- in wässrigem Medium löslichen Füllmitteln, beispielsweise Laktose,

- in wässrigem Medium unlöslichen, nicht quellenden Füllmitteln, beispielsweise Calciumhydrogenphosphat; und
- in wässrigem Medium unlöslichen, quellenden Füllmitteln, beispielsweise mikrokristalline Cellulose.

Bevorzugt ist das Füllmittel ausgewählt aus der Gruppe bestehend aus mikrokristalliner Cellulose, Calciumhydrogenphosphat und Laktose.

Die Freisetzungsprofile des Wirkstoffs, vorzugsweise (1R,2R)-3-(2-Dimethylaminomethyl-cyclohexyl)-phenol oder eines seiner pharmazeutisch verträglichen Salze, aus der erfindungsgemäßen Darreichungsform sind vorzugsweise unabhängig vom pH-Wert, wie er physiologisch während der Passage des Gastrointestinaltrakts auftreten kann. Die Freisetzungsprofile bei einem pH-Wert der Umgebung von 1,2 und 6,8 sind bevorzugt sowohl untereinander im Wesentlichen identisch als auch im Vergleich zur Freisetzung während eines Zeitprofils des pH-Werts von pH 1,2 über pH 2,3 und über pH 6,8 bis hin zu pH 7,2.

Die erfindungsgemäße Darreichungsform enthält als Wirkstoff 3-(2-Dimethylaminomethyl-cyclohexyl)-phenol oder eines seiner pharmazeutisch verträglichen Salze. Der Wirkstoff kann dabei als Gemisch zweier oder mehrerer seiner Stereoisomere (Enantiomere und/oder Diastereomere) vorliegen. In der erfindungsgemäßen Darreichungsform kann 3-(2-Dimethylaminomethyl-cyclohexyl)-phenol als Gemisch aller vier Diastereomere in beliebigem Mischungsverhältnis, aber auch als Gemisch von zwei oder drei der vier Stereoisomere oder in stereoisomerenreiner Form vorliegen. In einer bevorzugten Ausführungsform liegt der Wirkstoff als racemische Mischung des (1R,2R)/(1S,2S)-Enantiomerenpaars vor, wobei bevorzugt weder das (1R,2S)-, noch das (1S,2R)-Diastereomer enthalten ist oder deren Gewichtsanteil unterhalb von 2,0 Gew.-% liegt, bezogen auf das Gesamtgewicht des Wirkstoffs.

Pharmazeutisch verträgliche Salze des Wirkstoffs sind im Sinne dieser Erfindung solche Salze des Wirkstoffs, die bei pharmazeutischer Verwendung physiologisch - insbesondere bei Anwendung am Säugetier und/oder Menschen - verträglich sind. Solche pharmazeutisch verträglichen Salze können beispielsweise mit anorganischen oder organischen Säuren gebildet werden. Als Beispiele für Salze anorganischer Säuren können genannt werden: Hydrochloride, Hydrobromide, Sulfate, Phosphate, Hydrogenphosphate und Dihydrogenphosphate. Als Beispiele für Salze organischer Säuren können genannt werden: Formiate, Acetate, Propionate, Fumarate, Glutarate, Pyruvate, Malate, Tartrate, Benzoate, Citrate, Ascorbate, Maleate, etc.

In einer besonders bevorzugten Ausführungsform enthält die erfindungsgemäße Darreichungsform das Stereoisomer (1R,2R)-3-(2-Dimethylaminomethyl-cyclohexyl)-phenol oder eines seiner pharmazeutisch verträglichen Salze, wobei dessen Enantiomerenüberschuss vorzugsweise mindestens 90% ee, bevorzugter mindestens 95% ee, noch bevorzugter mindestens 97% ee und insbesondere mindestens 98% ee beträgt.

Der Wirkstoff 3-(2-Dimethylaminomethyl-cyclohexyl)-phenol kann als solches, d.h. als freie Base, aber auch in Form eines pharmazeutisch verträglichen Salzes, beispielsweise als Hydrochlorid, vorliegen. Die Herstellung der Hydrochloride ist beispielsweise aus DE-A 195 25 137 bekannt. Im Stand der Technik sind Verfahren bekannt, das Hydrochlorid in die freie Base oder in ein anderes pharmazeutisch verträgliches Salz zu überführen. Auch Methoden zur Trennung der Enantiomere bzw. Diastereomere sind im Stand der Technik hinreichend bekannt. Beispielsweise können die Diastereomere durch HPLC und die Enantiomere durch HPLC an chiralen stationären Phasen getrennt werden.

Die erfindungsgemäße Darreichungsform kann neben dem Wirkstoff 3-(2-Dimethylaminomethyl-cyclohexyl)-phenol bzw. einem seiner pharmazeutisch verträglichen Salze weitere pharmazeutisch wirksame Substanzen enthalten. Bevorzugt enthält die erfindungsgemäße Darreichungsform jedoch lediglich 3-(2-Dimethylaminomethyl-cyclohexyl)-phenol, vorzugsweise (1R,2R)-3-(2-Dimethylaminomethyl-cyclohexyl)-phenol oder eines seiner pharmazeutisch verträglichen Salze, und ansonsten keine weiteren pharmazeutisch wirksamen Substanzen.

Bevorzugte Ausführungsformen der erfindungsgemäßen Darreichungsform (Nr. 1 bis Nr. 5) weisen folgende Inhaltsstoffe in folgenden Mengen auf (die Prozentangaben sind jeweils bezogen auf das Gesamtgewicht der Darreichungsform):

| Inhaltsstoff | Gew.-% | | | | |
|---|---|---|---|---|---|
| | 1 | 2 | 3 | 4 | 5 |
| Wirkstoff, vorzugsweise (1R,2R)-3-(2-Dimethylaminomethyl-cyclohexyl)-phenol | 1,0-50 | 2,5-45 | 5,0-41 | 15-35 | 20-30 |
| Celluloseether oder Celluloseester, Wil MC, EC, HEC, HPC, CMC oder HPMC | 5,0-75 | 7,5-60 | 9,0-50 | 15-45 | 25-40 |
| Füllmittel, vorzugsweise mikrokristalline Cellulose, Calciumhydrogenphosphat oder Laktose | 10-90 | 20-75 | 30-66 | 35-60 | 40-55 |

(fortgesetzt)

| Inhaltsstoff | Gew.-% | | | | |
|---|---|---|---|---|---|
| | 1 | 2 | 3 | 4 | 5 |
| Fließregulierungsmittel, vorzugsweise hochdisperses Siliciumdioxid | 0-5,0 | 0-2,5 | 0-1,0 | 0,1-1,0 | 0,2-0,8 |
| Gleitmittel, vorzugsweise Magnesiumstearat | 0-5,0 | 0-2,5 | 0-1,0 | 0,1-1,0 | 0,2-0,8 |

Weitere Bestandteile der erfindungsgemäßen Darreichungsform können ggf. verdauliche, langkettige (d.h. mit 8 bis 50 C-Atomen, bevorzugt 12 bis 40 C-Atomen) unsubstituierte oder substituierte Kohlenwasserstoffe, wie z.B. Fettalkohole, Fettsäureglycerylester, Mineral- und Pflanzenöle sowie Wachse sein, wobei Kohlenwasserstoffe mit einem Schmelzpunkt zwischen 25°C und 90°C bevorzugt sind. Insbesondere sind Fettalkohole bevorzugt, ganz besonders Laurylalkohol, Myristylalkohol, Stearylalkohol, Cetylalkohol und Cetylstearylalkohol. Ihr Gehalt in der Darreichungsform beträgt bevorzugt 0 bis 20 Gew.-%.

Die erfindungsgemäße Darreichungsform ist durch vorteilhafte pharmakokinetische Parameter gekennzeichnet.

Zum Zwecke der Beschreibung sind die pharmakokinetischen Parameter, welche sich aus den Konzentrationen von 3-(2-Dimethylaminomethyl-cyclohexyl)-phenol im Blutplasma bestimmen lassen, wie folgt definiert:

| | |
|---|---|
| $\tau$ | Dosisintervall |
| $C_{max}$ | maximale, gemessene Plasmakonzentration des Wirkstoffs nach einmaliger Verabreichung (Ξ mittlerer *Peak-Plasma-Level*) |
| $C_{min}$ | minimale, gemessene Plasmakonzentration des Wirkstoffs nach einmaliger Verabreichung |
| $C_{av}$ | durchschnittliche Plasmakonzentration des Wirkstoffs nach einmaliger Verabreichung: $C_{av} = \frac{AUC_\tau}{\tau}$ |
| $t_{max}$ | Zeitspanne von der Verabreichung des Wirkstoffs bis zum Erreichen von $C_{max}$ |
| $t_{lag}$, t(0) | Zeitspanne von der Verabreichung bis zur ersten nachweisbaren Plasmakonzentration des Wirkstoffs (*lag-time*) |
| $AUC_\tau$ | Gesamtfläche der Plasmakonzentrations-Zeit-Kurve während $\tau$ |
| $AUC_{0-t}$ | Gesamtfläche der Plasmakonzentrations-Zeit-Kurve von der Verabreichung bis zum letzten Messwert |
| $AUC_{t-\infty}$ | Teilfläche der Plasmakonzentrations-Zeit-Kurve vom letzten Messwert extrapoliert bis zur Ewigkeit |
| AUC | Gesamtfläche der Plasmakonzentrations-Zeit-Kurve einschließlich der Teilfläche vom letzten Messwert extrapoliert bis zur Ewigkeit |
| $AUC\%_{extr}$ | Teilfläche der Plasmakonzentrations-Zeit-Kurve vom letzten Messwert extrapoliert bis zur Ewigkeit in Prozent |
| $\lambda_z$ | Geschwindigkeitskonstante der terminalen Eliminationsphase, definiert als die negative Steigung der Regressionsgeraden bei logarithmischer linearer Regression |
| $t_{1/2,z}$ | Halbwertszeit während der terminalen Eliminationsphase: $t_{1/2,z} = \frac{\ln(2)}{\lambda_z}$ |
| HVD | Halbwertsdauer, definiert als Zeitintervall, während dessen die Plasmakonzentration oberhalb von 50% von $C_{max}$ ist |
| MRT | mittlere Verweilzeit, definiert als Verhältnis von AUMC (Fläche unter der ersten Momentkurve "*first moment curve*") und AUC (vgl. M. Gibaldi, D. Perrier, J Pharm Sci. 1982, 71(4), 474-5) |
| CL/f | Gesamtclearance nach oraler Verabreichung: $CL/f = \frac{Dose}{AUC}$ |

(fortgesetzt)

| | |
|---|---|
| $\tau$ | Dosisintervall |
| $V_Z/f$ | apparentes Verteilungsvolumen während der terminalen Dispositions-phase nach oraler Verabreichung: $V_z/f = \frac{Dose}{AUC \bullet \lambda_z}$ |
| PTF | peak to through fluctuation über ein Verabreichungsintervall: $PTF\% = 100\frac{C_{max} - C_{min}}{C_{av}}$ |

Die vorstehenden Parameter werden jeweils als Durchschnittswerte über die Einzelwerte für alle untersuchten Patienten/Probanden angegeben.

Dem Fachmann ist bekannt, wie die pharmakokinetischen Parameter des Wirkstoffs 3-(2-Dimethylaminomethyl-cyclohexyl)-phenol aus den gemessenen Konzentrationen des Wirkstoffs im Blutplasma berechnet werden können. In diesem Zusammenhang kann beispielsweise verwiesen werden auf Cawello Willi (Editor) Parameters for Compatment-free Pharmacokinetics, Shaker Verlag Aachen (1999).

Bevorzugt wird nach vorzugsweise oraler Verabreichung der erfindungsgemäßen Darreichungsform *in vivo* der mittlere *Peak-Plasma-Level* ($C_{max}$) im Mittel nach $t_{max}$ 2 bis 10 h, bevorzugter nach 3 bis 8 h, noch bevorzugter nach 3,5 h bis 6 h, am bevorzugtesten nach 4,0 bis 5,5 h und insbesondere nach 4,2 bis 5,2 h erreicht.

Bevorzugt beträgt der mittlere Wert für MRT nach vorzugsweise oraler Verabreichung der erfindungsgemäßen Darreichungsform *in vivo* mehr als 7,5 h, bevorzugter mehr als 8,0 h, noch bevorzugter mehr als 9,0 h, am bevorzugtesten liegt er im Bereich von 10,0 bis 25,0 h und insbesondere im Bereich von 11,0 bis 20,0 h.

Bevorzugt beträgt der mittlere Wert für HVD nach vorzugsweise oraler Verabreichung der erfindungsgemäßen Darreichungsform *in vivo* mehr als 5,0 h, bevorzugter mehr als 6,0 h, noch bevorzugter mehr als 7,0 h, am bevorzugtesten liegt er im Bereich von 8,0 bis 20,0 h und insbesondere im Bereich von 9,0 bis 18,0 h.

Bevorzugt ist der mittlere Wert für $\lambda_z$ bei gleicher Dosis kleiner als bei einer Vergleichsformulierung ohne kontrollierte Freisetzung. Bevorzugt beträgt der mittlere Wert für $\lambda_z$ nach vorzugsweise oraler Verabreichung der erfindungsgemäßen Darreichungsform *in vivo* weniger als 0,125 $h^{-1}$, bevorzugter weniger als 0,122 $h^{-1}$, noch bevorzugter weniger als 0,118 $h^{-1}$, am bevorzugtesten liegt er im Bereich von 0,050 bis 0,115 $h^{-1}$ und insbesondere im Bereich von 0,060 bis 0,112 $h^{-1}$.

Bevorzugt ist der mittlere Wert für $t_{1/2,z}$ bei gleicher Dosis größer als bei einer Vergleichsformulierung ohne kontrollierte Freisetzung. Bevorzugt beträgt der mittlere Wert für $t_{1/2,z}$ nach vorzugsweise oraler Verabreichung der erfindungsgemäßen Darreichungsform *in vivo* mehr als 5,7 h, bevorzugter mehr als 6,0 h, noch bevorzugter mehr als 6,2 h, am bevorzugtesten liegt er im Bereich von 6,4 h bis 20,0 h und insbesondere im Bereich von 6,6 h bis 15,0 h.

Als "Vergleichsformulierung ohne kontrollierte Freisetzung" wird im Sinne der Beschreibung eine *immediate release* Formulierung des Wirkstoffs verstanden, beispielsweise ein Saft, z.B. eine Wirkstofflösung oder Wirkstoffdispersion mit gleicher Dosierung. In einer bevorzugten Ausführungsform wird darunter eine Wirkstofflösung verstanden, von der 1 ml folgende Bestandteile enthält:

| | |
|---|---|
| 10,0 mg | 3-(2-Dimethylaminomethyl-cyclohexyl)-phenol bzw. Äquivalentdosis eines seiner pharmazeutisch verträglichen Salze |
| 7,0 mg | Natriumchlorid, Ph.Eur., für parenterale Zwecke |
| 0,5 mg | Natriumcitrat · $2H_2O$, Ph.Eur., für parenterale Zwecke |
| 985,5 mg | Wasser für Injektionszwecke, Ph.Eur.* |

In einer anderen bevorzugten Ausführungsform wird unter "Vergleichsformulierung ohne kontrollierte Freisetzung" eine Kapselformulierung verstanden mit den Hilfsstoffen Mikrokristalline Cellulose, niedrig substituierte Hydroxypropylcellulose, Magnesiumstearat und Siliciumdioxid, vorzugsweise mit folgender Zusammensetzung:

| | |
|---|---|
| 30 mg | 3-(2-Dimethylaminomethyl-cyclohexyl)-phenol bzw. Äquivalentdosis eines seiner pharmazeutisch verträglichen Salze, |
| 231 mg | Mikrokristalline Cellulose, PH102, Ph.Eur., |
| 72 mg | niedrig substituierte Hydroxypropylcellulose, NF (L-HPC, Typ LH 11), |

(fortgesetzt)

| | |
|---|---|
| 18 mg | Magnesiumstearat, Ph.Eur., und |
| 9 mg | Hochdisperses Siliciumdioxid, Ph.Eur.; |

Gesamtgewicht: 360 mg, vorzugsweise in einer Hartgelatinekapsel Gr. 0el.

Bevorzugt beträgt bei der Dosis D des Wirkstoffs der mittlere Wert für $C_{max}/D$ nach vorzugsweise oraler Verabreichung der erfindungsgemäßen Darreichungsform *in vivo* $7{,}0\ 10^{-5}\ l^{-1} \leq C_{max}/D \leq 1{,}05\ 10^{-3}\ l^{-1}$, bevorzugter $8{,}0\ 10^{-5}\ l^{-1} \leq C_{max}/D \leq 1{,}0\ 10^{-3}\ l^{-1}$, noch bevorzugter $9{,}0\ 10^{-5}\ l^{-1} \leq C_{max}/D \leq 9{,}0\ 10^{-4}\ l^{-1}$, am bevorzugtesten $1{,}0\ 10^{-4}\ l^{-1} \leq C_{max}/D \leq 8{,}0\ 10^{-4}\ l^{-1}$, und insbesondere $2{,}0\ 10^{-4}\ l^{-1} \leq C_{max}/D \leq 7{,}0\ 10^{-4}\ l^{-1}$.

Bevorzugt beträgt der mittlere Wert für $C_{max}/AUC$ nach vorzugsweise oraler Verabreichung der erfindungsgemäßen Darreichungsform *in vivo* zwischen 0,150 und 0,010 $h^{-1}$, bevorzugter zwischen 0,125 und 0,020 $h^{-1}$, noch bevorzugter zwischen 0,100 und 0,030 $h^{-1}$, am bevorzugtesten zwischen 0,095 und 0,040 $h^{-1}$ und insbesondere zwischen 0,090 und 0,050 $h^{-1}$. Der Wert von $C_{max}/AUC$ kann als Surrogat für die Absorptionsgeschwindigkeit aufgefasst werden.

Bevorzugt beträgt bei zweimal täglicher Verabreichung, vorzugsweise im Absatnd von 12 h, der mittlere Wert für PTF < 80%, bevorzugter ≤ 75%, noch bevorzugter ≤ 70%, am bevorzugtesten ≤ 65% und insbesondere ≤ 60%.

Die erfindungsgemäße Darreichungsform enthält den Wirkstoff 3-(2-Dimethylaminomethyl-cyclohexyl)-phenol, vorzugsweise (1R,2R)-3-(2-Dimethylaminomethyl-cyclohexyl)-phenol, als solches und/oder als pharmazeutisch verträgliches Salz in einer Menge von üblicherweise 2,5 bis 800 mg, insbesondere 5 bis 400 mg, ganz besonders bevorzugt 10 bis 250 mg (Gewicht bezogen auf 3-(2-Dimethylaminomethyl-cyclohexyl)-phenol als Hydrochlorid) pro Dosierungseinheit, wobei das Freisetzungsverhalten der erfindungsgemäßen Darreichungsform durch die exakte Menge des Wirkstoffs praktisch nicht beeinflusst wird, solange die oben angegebenen Gehaltsgrenzen eingehalten werden.

In einer bevorzugten Ausführungsform der erfindungsgemäßen Darreichungsform ist diese zur oralen oder rektalen Verabreichung, vorzugsweise einmal oder zweimal täglich, konfektioniert. Bei täglich ein- oder zweimaliger Einnahme einer erfindungsgemäßen pharmazeutischen Darreichungsform durch den Patienten wird eine gute therapeutische Wirksamkeit bei anhaltend starken Schmerzen sicher erzielt.

Die erfindungsgemäßen pharmazeutischen Darreichungsformen können sowohl als einfache Tablette als auch als überzogene Tablette, beispielsweise als Filmtablette oder Drageé vorliegen. Üblicherweise sind die Tabletten rund oder bikonvex; möglich sind auch oblong Tablettenformen, die ggf. eine Teilbarkeit der Tablette gestatten. Ferner sind auch Granulate, Spheroide, Pellets oder Mikrokapseln möglich, die in Sachéts oder Kapseln gefüllt werden oder zu zerfallenden Tabletten verpresst werden können.

Zusätzlich zur verzögert freisetzenden Matrix in der pharmazeutischen Darreichungsform ist auch die Verwendung eines die Freisetzung des Wirkstoffs retardierenden Überzugs möglich. Dabei können der Wirkstoff - welcher vorzugsweise, jedoch nicht zwingend in einer Polymermatrix eingebettet ist - und ggf. weitere pharmazeutische Hilfsstoffe, wie etwa Bindemittel, Füllmittel, Gleit-, Schmier- und Fließregulierungsmittel, enthalten sein, die mit einem Material überzogen bzw. beschichtet werden, welches die verzögerte Freisetzung des Wirkstoffs in wässrigem Medium steuert und/oder moduliert. Geeignete Beschichtungsmittel sind z.B. wasserunlösliche Wachse und Polymere, wie Polymethacrylate (Eudragit o.ä.) oder wasserunlösliche Cellulosen, insbesondere Ethylcellulose. Ggf. können im Überzugsmaterial auch wasserlösliche Polymere, wie Polyvinylpyrrolidon, wasserlösliche Cellulosen, wie Hydroxypropylmethylcellulose oder Hydroxypropylcellulose, andere wasserlösliche Mittel, wie Polysorbat 80, oder hydrophile Porenbildner, wie Polyethylenglykol, Laktose oder Mannit, enthalten sein.

Für die überzogenen Darreichungsformen, vorzugsweise Tabletten, können ein oder mehrere Überzugsschichten verwendet werden. Als Überzugsmaterialien eignen sich bekannte Hydroxypropylmethylcellulosen mit niedriger Viskosität von ca. 1,0 bis 100 mPa·s und niedrigem Molekulargewicht von < 10.000 g $mol^{-1}$ (z.B. Pharmacoat 606 mit einer Viskosität von 6,0 mPa·s in einer 2,0 Gew.-%igen wässrigen Lösung bei 20°C), die das Freisetzungsprofil der erfindungsgemäßen Arzneimittel praktisch nicht oder nur geringfügig beeinflussen.

Dem Fachmann bekannte Diffusionsüberzüge, beispielsweise auf Basis von quellbaren, aber wasserunlöslichen Poly(meth)acrylaten, führen zu einer Modulation der Retardierung der Wirkstofffreisetzungen aus den erfindungsgemäßen pharmazeutischen Darreichungsformen. Der wirkstoffhaltige, den Wirkstoff retardiert freisetzende Kern mit einem Wirkstoffgehalt vorzugsweise zwischen 0,5 und 85 Gew.-%, besonders bevorzugt zwischen 3,0 und 70 Gew.-% und ganz besonders bevorzugt zwischen 8,0 und 66 Gew.-%, kann mit zusätzlichem Wirkstoff, der nicht retardiert, sondern als Initialdosis freigesetzt wird, durch verschiedene, dem Fachmann bekannte Verfahren, beispielsweise Dragieren, Aufsprühen aus Lösungen oder Suspensionen oder durch Pulverauftragverfahren, umhüllt sein, ohne dass dies für eine verzögerte Freisetzung bei gleichzeitigem raschen Anfluten des Wirkstoffs zur schnellen Schmerzlinderung bei erster Gabe der erfindungsgemäßen pharmazeutischen Formulierung zwingend erforderlich wäre.

Weitere Ausführungsformen der erfindungsgemäßen Darreichungsform stellen Mehrschicht- und Manteltabletten dar, bei denen der Wirkstoff in einer oder mehreren Schichten der Mehrschichttablette mit einem Wirkstoffgehalt

vorzugsweise zwischen 0,5 und 85 Gew.-%, besonders bevorzugt zwischen 3,0 und 70 Gew.-% und ganz besonders bevorzugt zwischen 8,0 und 66 Gew.-% bzw. im Kern der Manteltablette mit einem Wirkstoffgehalt vorzugsweise zwischen 0,5 und 85 Gew.-%, besonders bevorzugt zwischen 3,0 und 70 Gew.-% und ganz besonders bevorzugt zwischen 8,0 und 66 Gew.-% durch eine Polymermatrix retardiert freigesetzt wird und die Freisetzung des Wirkstoffs in einer oder mehreren Schichten der Mehrschichttablette bzw. der äußeren Mantelschicht der Manteltabletten unretardiert erfolgt. Mehrschicht - und Manteltabletten können ein oder mehrere wirkstofffreie Überzüge enthalten.

Die erfindungsgemäßen Darreichungsformen können beispielsweise nach folgendem allgemeinen Verfahren hergestellt werden:

> Die Bestandteile der Darreichungsform (Wirkstoff, Polymere zur Bildung der Polymermatrix [Matrixbildner] und fakultative Bestandteile) werden der Reihe nach eingewogen und anschließend auf einer üblichen Siebmaschine gesiebt. Hier kann beispielsweise die Siebmaschine *Quadro Comil U10* eingesetzt werden, wobei eine gebräuchliche Siebgröße ca. 0,813 mm beträgt. Die Siebung wird anschließend in einem Containermischer gemischt, z.B. in einem *Bohle* Containermischer, typische Arbeitsbedingungen sind: Dauer ca. 15 min ± 45 s bei einer Drehzahl von 20 ± 1 U/min. Danach wird die erhaltene Pulvermischung auf einer Tablettenpresse zu einer Tablette verpresst. Hierfür kann z.B. eine Tablettenpresse *Korsch EKO* mit einem drageegewölbten Rundstempel mit 10 mm Durchmesser Verwendung finden. Alternativ kann auch eine Kompaktierung der Pulvermischung und anschließende Siebung *(Comil* 3 mm Reibschnitzelsieb und anschließend 1,2 mm Rundlochsieb) der Presslinge erfolgen, wobei das so entstehende Granulat anschließend wie oben beschrieben unter Zusatz von Schmiermittel (z.B. Magnesiumstearat) auf z.B. einer *EKO* Tablettenpresse mit 10 mm Rundstempeln verpresst wird. Die Granulation kann auch durch Nassgranulation auf Basis wässriger oder organischer Lösungsmittel erfolgen; bevorzugt sind dabei wässriges Lösungsmittel mit oder ohne geeignete Bindemittel. Das Herstellungsverfahren kann ohne weiteres an die jeweiligen Erfordernisse und die gewünschte Darreichungsform nach im Stand der Technik wohlbekannten Vorgehensweisen angepasst werden.

Die Herstellung erfindungsgemäßer pharmazeutischer Darreichungsformen ist durch eine hohe Reproduzierbarkeit der Freisetzungseigenschaften der erhaltenen Zusammensetzungen, die 3-(2-Dimethylaminomethyl-cyclohexyl)-phenol oder eines seiner pharmazeutisch verträglichen Salze enthalten, gekennzeichnet. Während einer Lagerzeit von mindestens einem Jahr unter den üblichen Lagerungsbedingungen gemäß ICH Q1AR-*Stability-Testing-Guideline* erweist sich das Freisetzungsprofil erfindungsgemäßer Darreichungsformen als stabil.

Ein weiterer Gegenstand der Erfindung betrifft die Verwendung von 3-(2-Dimethylaminomethyl-cyclohexyl)-phenol oder eines seiner pharmazeutisch verträglichen Salze zur Herstellung einer vorstehend beschriebenen Darreichungsform zur Bekämpfung von Schmerz. Bevorzugt ist der Schmerz ausgewählt aus der Gruppe bestehend aus akutem Schmerz und chronischem Schmerz, insbesondere Entzündungsschmerz oder neuropathischem Schmerz, wobei der Schmerz schwach, mittelstark, stark oder stärkst sein kann.

Bevorzugt geht dabei die Bekämpfung von Schmerz mit einer signifikanten Verringerung der Nebenwirkung Übelkeit und/oder Erbrechen im Vergleich zu einer Darreichungsform ohne kontrollierte Freisetzung einher. Vorzugsweise erfolgt die Verabreichung oral.

Vorzugsweise ist der Schmerz ausgewählt aus akutem Schmerz und chronischem Schmerz.

Die folgenden Beispiele dienen der Illustration der vorliegenden Erfindung und bevorzugter Ausführungsformen

<u>Beispiel 1:</u>

Matrixtabletten mit folgender Zusammensetzung pro Tablette

| | mg | Gew.-% |
|---|---|---|
| (1R,2R)-3-(2-Dimethylaminomethyl-cyclohexyl)-phenol | 60 | 25 |
| Hydroxypropylmethylcellulose, 100.000 mPa·s | 100 | 41,7 |
| Mikrokristalline Cellulose (Avicel PH 102 von Fa. FA. FMC) | 77,5 | 32,3 |
| Hochdisperses Siliciumdioxid | 1,25 | 0,5 |
| Magnesiumstearat | 1,25 | 0,5 |
| Gesamtmenge | 240 | 100 |

wurden in einer Ansatzgröße von 1000 Tabletten in folgender Weise hergestellt:

Alle Bestandteile wurden eingewogen und auf einer Siebmaschine Quadro Comil U10 unter Verwendung einer *Siebgröße* von 0,813 mm gesiebt, in einem Containermischer (Bohle LM 40) 15 min ± 15 s bei einer Drehzahl von 20 ± 1 U/min gemischt und auf einer Korsch EKO Exzenterpresse zu drageegewölbten Tabletten mit einem Durchmesser von 10 mm, einem Wölbungsradius von 8 mm und einem mittleren Tablettengewicht von 240 mg gepresst.

Die Freisetzung *in vitro* wurde bestimmt unter Anwendung der Ph. Eur. Paddle Method bei 75 U/min in 900 ml Puffer pH 6,8 nach Ph. Eur. bei 37 °C und mit UV-spektrometrischem Nachweis und ist in folgender Tabelle wiedergegeben.

| Zeit [min] | Freigesetzte Gesamtmenge des Wirkstoffs [%] |
|---|---|
| 0 | 0 |
| 30 | 22 |
| 60 | 33 |
| 120 | 47 |
| 180 | 57 |
| 240 | 65 |
| 360 | 77 |
| 480 | 85 |
| 600 | 91 |
| 720 | 95 |

Beispiel 2:

Matrixtabletten mit folgender Zusammensetzung pro Tablette

| | A | | B | | C | | D | |
|---|---|---|---|---|---|---|---|---|
| | mg | Gew.-% | mg | Gew.-% | mg | Gew.-% | mg | Gew.-% |
| (1R,2R)-3-(2-Dimethylamino-methyl-cyclohexyl)-phenol | 15 | 6 | 40 | 16 | 80 | 32 | 100 | 40 |
| Hydroxypropylmethylcellulose, 100.000 mPa·s | 70 | 28 | 70 | 28 | 70 | 28 | 70 | 28 |
| Mikrokristalline Cellulose | 162,5 | 65 | 137,5 | 55 | 97,5 | 39 | 77,5 | 31 |
| Hochdisperses Siliciumdioxid | 1,25 | 0,5 | 1,25 | 0,5 | 1,25 | 0,5 | 1,25 | 0,5 |
| Maqnesiumstearat | 1,25 | 0,5 | 1,25 | 0,5 | 1,25 | 0,5 | 1,25 | 0,5 |
| Gesamtmenge | 250 | 100 | 250 | 100 | 250 | 100 | 250 | 100 |

wurden analog zu dem in Beispiel 1 angegebenen Verfahren hergestellt.

Die Freisetzung *in vitro* wurde wie in Beispiel 1 bestimmt.

| Zeit [min] | Freigesetzte Gesamtmenge des Wirkstoffs [%] | | | |
|---|---|---|---|---|
| | A | B | C | D |
| 0 | 0 | 0 | 0 | 0 |
| 30 | 27 | 23 | 20 | 19 |

(fortgesetzt)

| Zeit [min] | Freigesetzte Gesamtmenge des Wirkstoffs [%] | | | |
|---|---|---|---|---|
| | A | B | C | D |
| 60 | 37 | 33 | 32 | 31 |
| 120 | 51 | 48 | 48 | 47 |
| 180 | 60 | 58 | 60 | 59 |
| 240 | 68 | 68 | 69 | 69 |
| 360 | 81 | 81 | 82 | 83 |
| 480 | 90 | 89 | 90 | 92 |
| 600 | 96 | 95 | 95 | 96 |
| 720 | 100 | 100 | 98 | 99 |

Beispiel 3:

Matrixtabletten mit folgender Zusammensetzung pro Tablette

| A | B | C | mg | Gew.-% |
|---|---|---|---|---|
| (1R,2R)-3-(2-Dimethylaminomethylcyclohexyl)-phenol | (1R,2R)-3-(2-Dimethylaminomethylcyclohexyl)-phenol | (1R,2R)-3-(2-Dimethylaminomethylcyclohexyl)-phenol | 80 | 32 |
| Hydroxypropylmethylcellulose, 100.000 mPa·s | Hydroxypropylmethylcellulose, 100.000 mPa·s | Hydroxypropylmethyl cellulose, 100.000 mPa·s | 70 | 28 |
| Mikrokristalline Cellulose | Calciumhydrogen-phosphat | Laktose-Monohydrat | 97,5 | 39 |
| Hochdisperses Siliciumdioxid | Hochdisperses Siliciumdioxid | Hochdisperses Siliciumdioxid | 1,25 | 0,5 |
| Magnesiumstearat | Magnesiumstearat | Magnesiumstearat | 1,25 | 0,5 |
| Gesamtmenge | Gesamtmenge | Gesamtmenge | 250 | 100 |

wurden in einer Ansatzgröße von 75 Tabletten analog zu dem in Beispiel 1 angegebenen Verfahren hergestellt.
Die Freisetzung *in vitro* wurde wie in Beispiel 1 bestimmt.

| Zeit [min] | Freigesetzte Gesamtmenge des Wirkstoffs [%] | | |
|---|---|---|---|
| | A | B | C |
| 0 | 0 | 0 | 0 |
| 30 | 19 | 21 | 21 |
| 60 | 31 | 32 | 33 |
| 120 | 47 | 48 | 49 |
| 180 | 58 | 59 | 59 |
| 240 | 67 | 67 | 68 |
| 360 | 78 | 78 | 78 |
| 480 | 85 | 84 | 84 |
| 600 | 89 | 88 | 88 |
| 720 | 92 | 90 | 90 |

Beispiel 4:

Matrixtabletten mit folgender Zusammensetzung pro Tablette

| | mg | Gew.-% |
|---|---|---|
| (1R,2R)-3-(2-Dimethylaminomethyl-cyclohexyl)-phenol | 40 | 16 |
| Hydroxypropylmethylcellulose, 100.000 mPa·s | 70 | 28 |
| Mikrokristalline Cellulose | 137,5 | 55 |
| Hochdisperses Siliciumdioxid | 1,25 | 0,5 |
| Magnesiumstearat | 1,25 | 0,5 |
| Gesamtmenge | 250 | 100 |

wurden in einer Ansatzgröße von 100 Tabletten analog zu dem in Beispiel 1 angegebenen Verfahren hergestellt.
Die Freisetzung *in vitro* wurde unter folgenden Bedingungen bestimmt:

(A): wie in Beispiel 1 beschrieben;
(B): Anwendung der Ph. Eur. Paddle Method bei 75 U/min in 900 ml Puffer pH 1,2 nach USP 22 bei 37 °C und mit UV-spektrometrischem Nachweis;
(C): Anwendung der Ph. Eur. Paddle Method bei 75 U/min, wobei von 0-30 min ein pH von 1,2, von 30-120 min ein pH von 2,3, von 120-180 min ein pH von 6,5 und für die restliche Versuchszeit ein pH von 7,2 eingestellt wurde.

Die Tabelle gibt die Ergebnisse für die jeweiligen Versuchsbedingungen wieder:

| Zeit [min] | Freigesetzte Gesamtmenge des Wirkstoffs [%] | | |
|---|---|---|---|
| | A | B | C |
| 0 | 0 | 0 | 0 |
| 30 | 19 | 18 | 18 |
| 60 | 32 | 31 | 32 |

(fortgesetzt)

| Zeit [min] | Freigesetzte Gesamtmenge des Wirkstoffs [%] | | |
|---|---|---|---|
| | A | B | C |
| 120 | 48 | 46 | 47 |
| 180 | 60 | 59 | 60 |
| 240 | 68 | 67 | 67 |
| 360 | 79 | 78 | 77 |
| 480 | 87 | 86 | 84 |
| 600 | 92 | 92 | 90 |
| 720 | 95 | 95 | 94 |

<u>Beispiel 5:</u>

Matrixtabletten mit folgender Zusammensetzung pro Tablette

| | mg | Gew.-% |
|---|---|---|
| (1R,2R)-3-(2-Dimethylaminomethyl-cyclohexyl)-phenol | 80 | 32 |
| Hydroxypropylmethylcellulose 100.000 mPa·s | 70 | 28 |
| Mikrokristalline Cellulose | 97,5 | 39 |
| Hochdisperses Siliciumdioxid | 1,25 | 0,5 |
| Maqnesiumstearat | 1,25 | 0,5 |
| Gesamtmenge | 250 | 100 |

wurden in einer Ansatzgröße von 100 Tabletten analog zu dem in Beispiel 1 angegebenen Verfahren hergestellt. Die Verpressung erfolgte mit unterschiedlichen Presskräften, so das Tabletten mit folgenden Bruchfestigkeiten erhalten wurden: (A) 60 N, (B) 80 N, (C) 100 N, (D) 150 N.

Die Freisetzung *in vitro* wurde wie in Beispiel 1 bestimmt.

| Zeit [min] | Freigesetzte Gesamtmenge des Wirkstoffs [%] | | | |
|---|---|---|---|---|
| | A | B | C | D |
| 0 | 0 | 0 | 0 | 0 |
| 30 | 18 | 18 | 20 | 20 |
| 60 | 31 | 31 | 32 | 30 |
| 120 | 49 | 48 | 49 | 45 |
| 180 | 61 | 60 | 61 | 56 |
| 240 | 69 | 68 | 69 | 64 |
| 360 | 80 | 80 | 81 | 77 |
| 480 | 87 | 87 | 88 | 85 |
| 600 | 91 | 91 | 92 | 90 |
| 720 | 93 | 94 | 94 | 93 |

Beispiel 6:

Matrixtabletten mit folgender Zusammensetzung pro Tablette

| A | B | C | mg | Gew.-% |
|---|---|---|---|---|
| (1R,2R)-3-(2-Dimethylaminomethylcyclohexyl)-phenol | (1R,2R)-3-(2-Dimethylaminomethylcyclohexyl)-phenol | (1R,2R)-3-(2-Dimethylaminomethylcyclohexyl)-phenol | 40 | 16 |
| Hydroxypropylmethylcellulose, 90SH, 15.000 mPa·s | Hydroxypropylmethylcellulose, 60SH, 4.000 mPa·s | Hydroxypropylmethylcellulose, 65SH, 4.000 mPa·s | 70 | 28 |
| Mikrokristalline Cellulose | Calciumhydrogen-phosphat | Laktose-Monohydrat | 137,5 | 55 |
| Hochdisperses Siliciumdioxid | Hochdisperses Siliciumdioxid | Hochdisperses Siliciumdioxid | 1,25 | 0,5 |
| Maqnesiumstearat | Magnesiumstearat | Magnesiumstearat | 1,25 | 0,5 |
| Gesamtmenge | Gesamtmenge | Gesamtmenge | 250 | 100 |

wurden in einer Ansatzgröße von 200 Tabletten analog zu dem in Beispiel 1 angegebenen Verfahren hergestellt.

Die Freisetzung *in vitro* wurde wie in Beispiel 1 bestimmt.

| Zeit [min] | Freigesetzte Gesamtmenge des Wirkstoffs [%] | | |
|---|---|---|---|
| | A | B | C |
| 0 | 0 | 0 | 0 |
| 30 | 19 | 22 | 21 |
| 60 | 34 | 35 | 33 |
| 120 | 52 | 51 | 47 |
| 180 | 65 | 62 | 57 |
| 240 | 73 | 69 | 65 |
| 360 | 83 | 79 | 75 |
| 480 | 90 | 84 | 82 |
| 600 | 92 | 88 | 86 |
| 720 | 94 | 90 | 89 |

Beispiel 7:

Matrixtabletten mit folgender Zusammensetzung pro Tablette

| [mg] | mg | Gew.-% |
|---|---|---|
| (1R,2R)-3-(2-Dimethylaminomethyl-cyclohexyl)-phenol Maleat | 209,64 | 38,12 |
| Hypromellose 15.000 mPas | 60,00 | 10,91 |
| Lactose Typ 200 | 268,36 | 48,79 |
| Kolloidales wasserfreies Siliciumdioxid | 6,00 | 1,09 |
| Magnesiumstearat | 6,00 | 1,09 |
| Gewicht des Tablettenkems | 550,00 | 100,00 |

wurden in einer Ansatzgröße von 1.000 Tabletten hergestellt. Dazu wurden alle Bestandteile der Tablette eingewogen, durch ein 0,315 mm Sieb gesiebt, gemischt, mit Wasser in einem Kenwood Mischer granuliert, durch ein 1 mm Sieb getrieben, bei 50°C in einem Hoirdentrockenschrank getrocknet und auf einer Korsch EKO Exzentertablettenpresse mit 7 x 17 mm Oblongstempeln mit einem Gewicht von 550 mg pro Tablette verpresst.

Die Tabletten wiesen *in vitro* folgende Freisetzungen auf:

| Zeit [min] | Freigesetzte Gesamtmenge des Wirkstoffs [%] |
|---|---|
| 0 | 0 |
| 30 | 16 |
| 240 | 61 |
| 480 | 92 |

Beispiel 8:

a) Filmtabletten mit folgender Zusammensetzung pro Tablette

| [mg] | PR (A) | PR (B) | Ref. PR (C) |
|---|---|---|---|
| (1R,2R)-3-(2-Dimethylaminomethyl-cyclohexyl)-phenol | 60,00 | 60,00 | 60,00 |
| Hypromellose 100.000 mPas (Shin-Etsu) | 50,00 | 100,00 | 200,00 |
| Mikrokristalline Cellulose (Avicel 102, FMC) | 137,50 | 87,50 | 85,00 |
| Hochdisperses Siliciumdioxid | 1,25 | 1,25 | 2,50 |
| Magnesiumstearat | 1,25 | 1,25 | 2,50 |
| Gewicht des Tablettenkems | 250,00 | 250,00 | 350,00 |

wurden in einer Ansatzgröße von 1.000 Tabletten hergestellt. Dazu wurden alle Bestandteile des Tablettenkems eingewogen, durch ein 0,315 mm Sieb gesiebt, gemischt und auf einer Fette P1200 Rundläufertablettenpresse mit 10 mm Stempeln und einem Wölbungsradius von 8 mm verpresst. Anschließend wurden die Tabletten (Formulierung A und B) mit einer wässrigen Lacksuspension (ca. 29% Feststoffgehalt) aus Hypromellose 6 mPas, Macrogol 6000, Propylenglykol, Talkum, Titandixid und Gereinigtem Wasser befilmt, bis das Tablettengewicht um 12 mg zugenommen hatte.

Die Filmtabletten wiesen *in vitro* folgende Freisetzungswerte auf:

| Zeit [min] | Freigesetzte Gesamtmenge des Wirkstoffs [%] | | |
|---|---|---|---|
| | PR (A) | PR (B) | Ref. PR (C) |
| 0 | 0 | 0 | 0 |
| 30 | 22 | 18 | 14 |
| 180 | 65 | | |
| 240 | | 62 | 47 |
| 360 | 85 | | |
| 480 | | 84 | |
| 720 | | | 80 |

Wir vorstehende Tabelle zeigt, erfolgte eine Freisetzung von 80% des Wirkstoffs bei den Formulierungen PR (A), PR (B) und PR (C) *in vitro* nach ca. 360 min, 480 min bzw. 720 min.

b) In einer offenen, randomisierten Phase 1 vierfach-Crossover-Studie wurden die pharmakokinetischen Parameter für diese zwei erfindungsgemäßen Darreichungsformen mit unterschiedlichem Freisetzungsverhalten [*prolonged release,* PR (A) und PR (B) Sowie hür das Referenz beispiel PR (C) *in vivo* bestimmt und mit einer Wirkstofflösung [*immediate release,* [R] als Referenzformulierung verglichen. 1 ml der IR Wirkstofflösung enthielt:

| | |
|---|---|
| 10,0 mg | (1R,2R)-3-(2-Dimethylaminomethyl-cyclohexyl)-phenol |
| 7,0 mg | Natriumchlorid, Ph.Eur., für parenterale Zwecke |
| 0,5 mg | Natriumcitrat · $2H_2O$, Ph.Eur., für parenterale Zwecke |
| 985,5 mg | Wasser für Injektionszwecke, Ph.Eur.* |

Die Herstellung erfolgte nach der Standardprozedur für Injektionslösungen; die Abfüllung erfolgte zu 1 ml in Ampullen.

Es wurden jeweils eine Dosis D von 60 mg (1R,2R)-3-(2-Dimethylaminomethyl-cyclohexyl)-phenol an 8 freiwillige Probandinnen verabreicht und die Plasmakonzentration des Wirkstoffs über 32 Stunden gemessen.

Die quantitative Analyse der Konzentration von (1R,2R)-3-(2-Dimethylaminomethyl-cyclohexyl)-phenol im Blutplasma erfolgte unter Verwendung von O-Desmethyltramadol (M1) als interner Standard. Die Wirkstoffe wurden durch Flüssig-Flüssig-Extraktion mit *ter.*-Butylmethylether aus den Proben extrahiert. Die Extrakte wurden mit HPLC durch fluorometrische Detektion analysiert. Die Eichungskurven zeigten im Bereich der Plasmakonzentration von 0,23 ng/ml bis 92 ng/ml Linearität der Signale. Die Ergebnisse sind in Figuren 1A und 1B dargestellt. Figur 1A zeigt eine lineare Darstellung der Plasmakonzentration (Ordinate), Figur 1 B eine logarithmische Darstellung.

Die Berechnung der pharmakokinetischen Parameter erfolgte durch unkompartimentierte Analyse unter Verwendung des validierten Software-Programs MODUNA, welches wurde von der Grünenthal GmbH entwickelt wurde.

Die Berechnungen erfolgten mit allen verfügbaren Nachkommastellen ohne Rundungen.

Zur Berechnung der ersten Teilfläche von der Verabreichung bis zur ersten validen Plasmakonzentration wurde in Abhängigkeit von der Art der Verabreichung ein Konzentrationswert $C_{tdose}$ extrapoliert bis zur Verabreichungszeit tdose. War $t_{lag}$ > 0 h, so wurde die erste Teilfläche ab $t_{lag}$ berechnet.

AUC wurde durch Summierung berechnet aus der Teilfläche $AUC_{0-t}$ (Fläche unter der Konzentrations-Zeit-Kurve mit gemessenen Konzentrationen oberhalb der Nachweisgrenze) und der Restfläche $AUC_{t-\infty}$ (= $\hat{c}_t/\lambda_z$), worin $\hat{c}_t$ die auf Grundlage logarithmischer linearer Regression geschätzte Plasmakonzentration ist zum Zeitpunkt der letzten Messung, an dem noch eine Plasmakonzentration oberhalb der Nachweisgrenze bestimmt wurde.

$AUC_{0-t}$ wurde durch numerische Integration unter Anwendung der Trapezformel (Gibaldi und Perrier, 1982) berechnet. Bis $C_{max}$ wurde die lineare Trapezformel angewendet, danach die logarithmische Trapezformel. Wurden mehrere Maxima der Plasmakonzentration beobachtet, so wurde $C_{max}$ für das erste Maximum bestimmt.

Die Geschwindigkeitskonstante der terminalen Eliminationsphase $\lambda_z$ wurde durch logarithmische lineare Regression der terminalen Phase der Plasmakonzentrationskurven bestimmt (Cawello, 1999). Die $\lambda_z$ Zeitintervalle [h] für die Regression wurden gemäß nachfolgender Tabelle festgelegt:

| Probandin | 1 | 2 | 3 | 4 | 5 | 6 | 7 | 8 |
|---|---|---|---|---|---|---|---|---|
| PR(A) | 10-32 | 10-32 | 10-32 | 10-32 | 10-32 | 10-32 | 10-32 | 10-32 |
| PR(B) | 10-32 | 10-32 | 10-32 | 10-32 | 10-32 | 10-32 | 10-32 | 10-32 |
| PR(C) | 16-32 | 10-28 | 10-32 | 10-32 | 10-32 | 13-32 | 13-32 | 10-32 |
| IR | 20-32 | 10-32 | 10-32 | 10-32 | 10-32 | 10-32 | 20-32 | 13-28 |

Bei der Bestimmung der Halbwertsdauer HVD wurde der Zeitpunkt, an dem die Plasmakonzentration 50% von $C_{max}$ betrug, durch lineare Interpolation bestimmt.

Die aus den gemessenen Plasmakonzentrationen berechneten pharmakokinetischen Parameter sind in nachfolgender Tabelle zusammengefasst:

| D = 60 mg | PR (A) | | PR (B) | | Ref. PR (C) | | IR [*Vergleich*] | |
|---|---|---|---|---|---|---|---|---|
| | Mittel | CV [%] | Mittel | CV [%] | Mittel | CV [%] | Mittel | CV [%] |
| $t_{max}$ [h] | 5,00 | 18,5 | 4,50 | 23,8 | 4,75 | 27,0 | 1,25 | 37,0 |
| $t_{1/2,z}$ [h] | 6,68 | 23,5 | 7,44 | 27,7 | 12,10 | 32,5 | 5,69 | 25,0 |
| MRT [h] | 11,75 | 11,1 | 13,47 | 19,1 | 19,59 | 28,5 | 7,28 | 16,7 |
| HVD [h] | 9,40 | 23,1 | 12,05 | 24,5 | 15,07 | 39,0 | 4,78 | 42,8 |
| $\lambda_z$ [$h^{-1}$] | 0,109 | 23,4 | 0,099 | 26,2 | 0,063 | 33,9 | 0,128 | 22,2 |
| V(z)/f[l] | 1.569 | 40,8 | 1.665 | 29,5 | 2.817 | 52,7 | 1.102 | 26,1 |
| CL/f [ml $min^{-1}$] | 2.708 | 34,9 | 2.747 | 44,0 | 2.800 | 54,7 | 2.392 | 43,7 |
| $AUC_{0-t}$ [h nq $ml^{-1}$] | 343 | 39,3 | 341 | 41,7 | 310 | 42,9 | 404 | 32,5 |
| AUC [h ng $ml^{-1}$] | 359 | 38,6 | 369 | 41,4 | 384 | 44,3 | 411 | 33,0 |
| $C_{max}$ [ng $ml^{-1}$] | 32,2 | 58,3 | 26,8 | 65,6 | 19,7 | 38,0 | 64,2 | 40,9 |
| $C_{max}$/D [$l^{-1}$] | 5,37 $10^{-4}$ | - | 4,48 $10^{-4}$ | - | 3,28 $10^{-4.}$ | - | 1.07 $10^{-3}$ | - |
| $C_{max}$/AUC [$h^{-1}$] | 0,090 | - | 0.073 | - | 0,051 | - | 0,156 | - |

Die retardierten Formulierungen (*prolonged* release PR (A), PR (B) und Ref. PR (C)), welche unter *in vitro* Bedingungen 80% des Wirkstoffs nach ca. 360 min, 480 min bzw. 720 min freisetzten, zeigten auch *in vivo* beim Menschen eine zunehmende retardierende Wirkung.

Während der Mittelwert von $C_{max}$ bei Verabreichung der Wirkstofflösung 64,2 ng/ml betrug (Vergleich, *immediate release,* IR), nahm er bei Verabreichung der erfindungsgemäßen Formulierungen auf 32,2 ng/ml (PR(A)), 26,8 ng/ml (PR(B)), bzw. 19,7 ng/ml (Ref. PR(C)) ab.

Der Mittelwert von $t_{max}$ war bei beiden erfindungsgemäßen Formulierungen ähnlich und lag im Bereich von 4,5 bis 5,0 h, jedoch im Vergleich zur Verabreichung der Wirkstofflösung (1,25 h) eindeutig verzögert.

Die mittlere Halbwertsdauer HVD nahm von 4,78 h bei Verabreichung der Wirkstofflösung auf 9,40 h (PR (A)), 12,05 (PR (B)) bzw. 15,07 h (Ref. PR (C)) zu. Die Ergebnisse für die mittlere Verweilzeit bestätigten diesen Trend mit Zunahmen von 7,28 h bei Verabreichung der Wirkstofflösung auf bis zu 19,59 h (Ref. PR (C)).

Die mittlere Halbwertszeit während der terminalen Eliminationsphase nahm von 5,69 h bei Verabreichung der Wirkstofflösung auf 6,68 h (PR (A)), 7,44 h (PR (B)) bzw. 12,10 h (Ref. PR (C)) zu. Eine ausreichend retardierende Wirkung ist im Vergleich zu einer *immediate release* Formulierung (Wirkstofflösung, Saft, IR) durch Halbierung von $C_{max}$ oder Verdoppelung von HVD bei gleichzeitiger Zunahme von $t_{1/2,z}$ gekennzeichnet, im Idealfall ohne dabei AUC zu verändern.

In einer Studie lagen die PTF% Einzelwerte nach zweimal täglicher Gabe zwischen 49% und 88% in einem Dosisbereich von 160 bis 400 mg Tagesdosis. Die Mittelwerte innerhalb der Dosisgruppen lagen zwischen 57% und 64%.

c) In klinischen Prüfungen wurde gefunden, dass im Vergleich zu herkömmlichen Darreichungsformen zur oralen Verabreichung von 3-(2-Dimethylaminomethyl-cyclohexyl)-phenol Nebenwirkungen, insbesondere Übelkeit und/ oder Erbrechen, signifikant reduziert werden können.

Dies hat u.a. den Vorteil, dass sich die therapeutische Breite (Verhältnis der therapeutischen zur toxischen Wirkstoffdosis) von 3-(2-Dimethylaminomethyl-cyclohexyl)-phenol erhöht.

## Patentansprüche

1. Darreichungsform zur kontrollierten Freisetzung des Wirkstoffs 3-(2-Dimethylaminomethyl-cyclohexyl)-phenol oder eines seiner pharmazeutisch verträglichen Salze, welche

   - *in vitro,* gemessen gemäß Europäischem Arzneibuch mit einer Blattrührapparatur in Puffer bei einem pH-Wert von 6,8, einer Temperatur von 37°C und 75 U/min,

     - nach 0,5 Stunden 9,0 bis 31 Gew.-%,
     - nach 1 Stunde 20 bis 48 Gew.-%,
     - nach 2 Stunden 31 bis 65 Gew.-%,
     - nach 3 Stunden 42 bis 76 Gew.-%,
     - nach 4 Stunden 50 bis 83 Gew.-%,
     - nach 6 Stunden 60 bis 94 Gew.-%,
     - nach 8 Stunden 71 bis 99 Gew.-%,
     - nach 12 Stunden mehr als 79 Gew.-%,

     des ursprünglich in der Darreichungsform enthaltenen Wirkstoffs freisetzt,

   - eine Polymermatrix umfasst, welche auf einem Celluloseether oder Celluloseester basiert, der bei einer Konzentration von 2,0 Gew.-% in einer wässrigen Lösung bei 20°C eine Viskosität im Bereich von 3.000 bis 150.000 mPa·s aufweist,

   wobei zumindest ein Teil des Wirkstoffs in der Polymermatrix eingebettet ist,
   wobei der Gewichtsanteil der Polymermatrix 5 bis 85 Gew.-%, bezogen auf das Gesamtgewicht der Darreichungsform, beträgt, und
   wobei der Celluloseether bzw. Celluloseester ausgewählt ist aus der Gruppe bestehend aus Methylcellulose, Ethylcellulose, Hydroxyethylcellulose, Hydroxypropylcellulose, Carboxymethylcellulose und Hydroxypropylmethylcellulose.

2. Darreichungsform nach Anspruch 1, **dadurch gekennzeichnet, dass** es sich bei dem Wirkstoff um (1R,2R)-3-(2-Dimethylaminomethyl-cyclohexyl)-phenol oder eines seiner pharmazeutisch verträglichen Salze handelt.

3. Darreichungsform nach Anspruch 1 oder 2, **dadurch gekennzeichnet, dass** sie in Form einer Tablette vorliegt.

**4.** Verwendung des Wirkstoffs 3-(2-Dimethylaminomethyl-cyclohexyl)-phenol oder eines seiner pharmazeutisch verträglichen Salze zur Herstellung einer Darreichungsform nach einem der Ansprüche 1 bis 3 zur Bekämpfung von Schmerz.

**5.** Verwendung nach Anspruch 4, **dadurch gekennzeichnet, dass** die Verabreichung oral erfolgt.

## Claims

**1.** Dosage form for controlled release of the active ingredient 3-(2-dimethylamino-methylcyclohexyl)phenol or one of the pharmaceutically acceptable salts thereof, which

- *in vitro,* measured in accordance with the European Pharmacopoeia with a paddle stirrer apparatus in buffer at a pH value of 6.8, a temperature of 37°C and 75 rpm, releases

  - after 0.5 hour 9.0 to 31 % by weight,
  - after 1 hour 20 to 48% by weight,
  - after 2 hours 31 to 65% by weight,
  - after 3 hours 42 to 76% by weight,
  - after 4 hours 50 to 83% by weight,
  - after 6 hours 60 to 94% by weight,
  - after 8 hours 71 to 99% by weight,
  - after 12 hours more than 79% by weight

of the active ingredient originally contained in the dosage form,
- comprises a polymer matrix based on a cellulose ether or cellulose ester which at a concentration of 2.0% by weight in an aqueous solution at 20°C has a viscosity in the range of 3 000 to 150 000 mPa·s,
wherein at least a portion of the active ingredient is embedded in the polymer matrix,
wherein the proportion by weight of the polymer matrix amounts to 5 to 85% by weight, relative to the total weight of the dosage form, and
wherein the cellulose ether or the cellulose ester is selected from the group consisting of methylcellulose, ethylcellulose, hydroxyethylcellulose, hydroxypropylcellulose, carboxymethylcellulose and hydroxypropylmethylcellulose.

**2.** Dosage form according to claim 1, **characterised in that** the active ingredient is (1R,2R)-3-(2-dimethylaminomethylcyclohexyl)phenol or one of the pharmaceutically acceptable salts thereof.

**3.** Dosage form according to claim 1 or 2, **characterised in that** it is provded in the form of a tablet.

**4.** Use of the active ingredient 3-(2-dimethylaminomethylcyclohexyl)phenol or one of the pharmaceutically acceptable salts thereof for producing a dosage form according to one of claims 1 to 3 for combating pain.

**5.** Use according to claim 4, **characterised in that** administration proceeds orally.

## Revendications

**1.** Forme pharmaceutique pour la libération contrôlée de l'agent actif 3-(2-diméthylaminométhyl-cyclohexyl)-phénol ou d'un de ses sels pharmaceutiquement acceptables, qui

- libère *in vitro,* mesuré selon la Pharmacopée européenne avec un appareil d'agitation à pales dans un tampon à un pH de 6,8, à une température de 37 °C et à 75 tours/min,

  - après 0,5 heure, 9,0 à 31 % en poids,
  - après 1 heure, 20 à 48 % en poids,
  - après 2 heures, 31 à 65 % en poids,
  - après 3 heures, 42 à 76 % en poids,
  - après 4 heures, 50 à 83 % en poids,

- après 6 heures, 60 à 94 % en poids,
- après 8 heures, 71 à 99 % en poids,
- après 12 heures, plus de 79 % en poids,

de l'agent actif initialement contenu dans la forme pharmaceutique,
- comprend une matrice polymère, à base d'un éther de cellulose ou d'un ester de cellulose, qui présente à une concentration de 2,0 % en poids dans une solution aqueuse à 20 °C une viscosité dans la plage allant de 3 000 à 150 000 mPa·s,

au moins une partie de l'agent actif étant incorporé dans la matrice polymère,
la proportion en poids de la matrice polymère étant de 5 à 85 % en poids, par rapport au poids total de la forme pharmaceutique, et
l'éther de cellulose ou l'ester de cellulose étant choisi dans le groupe constitué par la méthylcellulose, l'éthylcellulose, l'hydroxyéthylcellulose, l'hydroxypropylcellulose, la carboxyméthylcellulose et l'hydroxypropylméthylcellulose.

**2.** Forme pharmaceutique selon la revendication 1, **caractérisée en ce que** l'agent actif est le (1R,2R)-3-(2-diméthylaminométhyl-cyclohexyl)-phénol ou un de ses sels pharmaceutiquement acceptables.

**3.** Forme pharmaceutique selon la revendication 1 ou 2, **caractérisée en ce qu'**elle se présente sous la forme d'un comprimé.

**4.** Utilisation de l'agent actif 3-(2-diméthylaminométhyl-cyclohexyl)-phénol ou d'un de ses sels pharmaceutiquement acceptables pour la fabrication d'une forme pharmaceutique selon l'une quelconque des revendications 1 à 3 pour lutter contre la douleur.

**5.** Utilisation selon la revendication 4, **caractérisée en ce que** l'administration a lieu par voie orale.

# Figur 1A

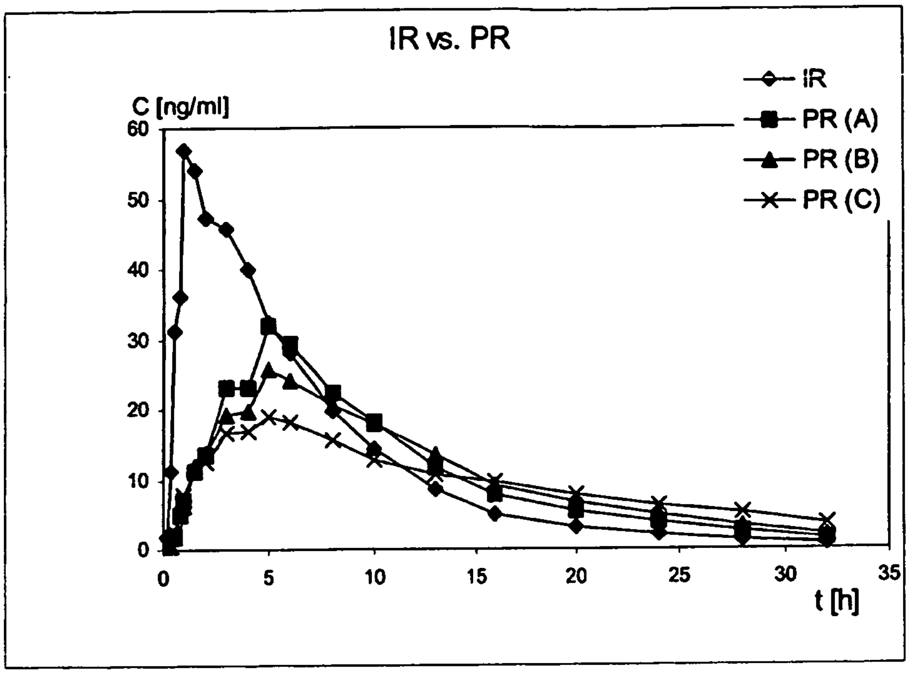


# Figur 1B

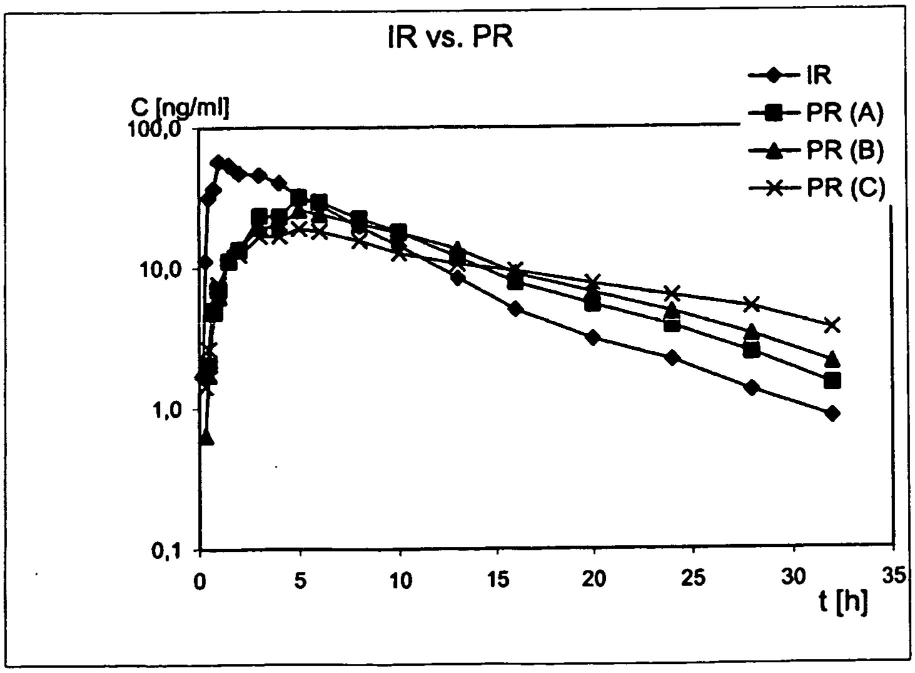

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19525137 A
- WO 0243712 A
- WO 0267916 A
- WO 02067651 A
- WO 2005009329 A
- US 5601842 A
- DE 4329794


### In der Beschreibung aufgeführte Nicht-Patentliteratur


- Lexikon der Hilfsstoffe für Pharmazie. **H.P. FIEDLER.** Kosmetik und angrenzende technische Gebiete. 2002
- **PERRIER.** *J Pharm Sci.,* 1982, vol. 71 (4), 474-5
- Parameters for Compatment-free Pharmacokinetics. Shaker Verlag Aachen, 1999